# FASCICULE DE BREVET EUROPEEN

(11) **EP 3 731 770 B1**
(45) Date de publication et mention de la délivrance du brevet: **13.10.2021**
(21) Numéro de dépôt: 18833673.9
(22) Date de dépôt: 27.12.2018
(51) Int. Cl.: A61B 18/02, A61B 17/00, A61B 18/00

(54) **BUSE D'APPLICATION POUR DISPOSITIF DE TRAITEMENT DERMO-COSMÉTIQUE DES TACHES BRUNES CUTANÉES PAR CRYOTHÉRAPIE CYTO-SÉLECTIVE**
APPLIKATIONSSDÜSE FÜR EIN DERMO-KOSMETISCHES GERÄT ZUR BEHANDLUNG BRAUNER HAUTFLECKEN MITTELS CYTO-SELEKTIVER KRYOTHERAPIE
APPLICATION NOZZLE FOR DERMO COSMETIC TREATMENT DEVICE FOR CUTANEOUS BROWN SPOTS BY CYTO-SELECTIVE CRYOTHERAPY

(30) Priorité: 28.12.2017 FR 1771438
(43) Date de publication de la demande: 04.11.2020
(73) Titulaire: CRYONOVE PHARMA, 28000 Chartres (FR)
(72) Inventeur: MARIN, Denis, 78620 L' Etang la ville (FR); ANTON, Jean-Christophe, 67000 Strasbourg (FR)
(74) Mandataire: Alatis
(86) Numéro de dépôt international: PCT/EP2018/097051
(87) Numéro de publication internationale: WO 2019/129827

(56) Documents cités:
- FR-A1- 2 913 960
- US-A1- 2004 102 768
- US-A1- 2007 005 048
- US-A1- 2017 354 451

## Description

L'invention se rapporte à un perfectionnement du dispositif de traitement dermo-cosmétique des taches brunes cutanées par cryothérapie cyto-sélective.

Plus précisément, l'invention concerne une buse perfectionnée destinée à être intégrée à un dispositif de traitement cosmétique des taches brunes cutanées par cryothérapie ainsi que le dispositif de traitement équipé de ladite buse.

### EXPOSÉ DE L'INVENTION

Le dispositif de traitement concerné par l'invention vise, plus particulièrement, le traitement dermo-cosmétique des taches brunes situées au niveau des mains, du visage, des membres et du décolleté de sujets atteints de telles hyper-pigmentations cutanées.

Un tel dispositif est décrit dans le WO2016113305 et comprend un réservoir de fluide cryogénique sous pression, une électrovanne permettant l'éjection du fluide depuis l'intérieur du réservoir vers un gicleur qui conduit, via une buse, le fluide sur la zone de la peau à traiter.

Le gicleur comprend au moins un conduit interne axial et cylindro-conique formant moyen de limitation du débit de fluide cryogénique.

L'électrovanne est associée à un système électronique de temporisation permettant son ouverture pendant une durée prédéterminée.

La transition de la phase liquide à la phase gazeuse, par détente du fluide cryogénique, s'opère au niveau de la buse qui est destinée à produire l'effet cryogénique recherché en étant en contact avec la zone cible de la peau, c'est-à-dire la zone sur laquelle se trouvent une tache brune à faire disparaître.

La buse est donc l'un des composants essentiels du dispositif de traitement cosmétique car elle conditionne le potentiel cryogénique du gaz et son impact sur la zone ciblée de l'épiderme et influe donc directement sur l'efficacité du traitement.

Le document US2004/102768A1 décrit un dispositif de traitement cosmétique par cryothérapie comprenant, notamment, une buse pourvue d'une bague supérieure prolongée par un corps central dont la paroi latérale présente, en partie basse, des ouvertures longitudinales et délimite une enceinte de détente d'un gaz cryogénique qui communique avec un conduit de dispersion ménagé à l'intérieur d'un embout d'application et qui débouche à l'extérieur par un orifice d'éjection.

Par ailleurs, le traitement dermo-cosmétique impliquant plusieurs applications successives du fluide cryogénique sur la peau, son efficacité dépend des conditions de mise en œuvre du fluide qui doivent être reproductibles de façon très précise.

Plus précisément, du fait que le fluide cryogénique passe d'une phase liquide à une phase gazeuse avant d'arriver sur sa cible, les caractéristiques de la buse d'application, qui est le siège de cette transition, sont donc importantes pour obtenir une courbe de température optimale.

Le suivi de cette courbe de température, dite de référence, permet de créer un choc thermique puis osmotique impactant les mélanocytes tout en préservant les autres cellules de l'épiderme et du derme et donc en conservant le caractère cyto-sélectif du traitement dermo-cosmétique.

De manière qualitative, cette courbe de température de référence comprend une descente rapide en température jusqu'à atteindre un palier délimité par une plage de températures, le maintien de la température dans cette plage de températures pendant une durée déterminée, avant d'effectuer une remontée progressive en température, jusqu'à revenir à sa valeur de départ.

Par conséquent, le passage du fluide cryogénique d'une phase liquide à une phase gazeuse en suivant la courbe de référence est sous l'influence de plusieurs paramètres, dont :
- le volume de l'enceinte de détente du gaz ainsi que la surface relative de ses ouvertures de communication avec l'extérieur et donc le profil de la buse,
- la distance entre la sortie du gicleur et la zone cible de la peau sur laquelle va s'appliquer l'effet cryogénique et donc la hauteur de la buse et, en particulier, celle de l'embout d'application qui est disposé au débouché de l'enceinte de détente.

En effet, le profil de cette buse s'avère techniquement très important car l'effet cryogénique recherché engendre la formation d'eau cristallisée ou de givre sur la zone cible de l'épiderme , ce qui permet de maintenir stable une température négative pendant une durée déterminée. Ainsi, une buse de forme trop plate ne permettrait pas de maintenir assez longtemps la température dans la plage préférentielle alors qu'une forme trop profonde entraînerait une durée trop longue de contact entre la cible et l'eau cristallisée, engendrant des températures trop basses.

Dans ces dernières conditions, la courbe de température obtenue ne pourrait pas correspondre à la courbe de référence.

Parallèlement, si la hauteur et/ou la surface des ouvertures de la buse sont trop faibles, le gaz n'a pas la possibilité de se détendre et se trouve projeté sur la zone cible sous la forme d'un mélange de gouttelettes et de gaz. Cette phase liquide s'accumule alors sur la zone cible, se détend progressivement et engendre ainsi des variations de températures incontrôlées et non reproductibles.

Dans le cas inverse, le gaz se disperse entièrement dans le volume intérieur de la buse et ne peut donc pas apporter au traitement tout son potentiel cryogénique.

Dans ces deux situations, la courbe de température obtenue sur la zone à traiter s'écarte notablement de la courbe de référence correspondant à un traitement dermo-cosmétique optimal.

La buse de l'invention vise à atteindre les objectifs de performance définis ci-dessus en produisant un effet cryogénique reproductible et homogène sur la zone à traiter sans modifier les propriétés cryogéniques intrinsèques du gaz et à permettre ainsi un traitement dermo-cosmétique efficace.

Ce but est atteint selon l'invention au moyen d'une buse, caractérisée en ce qu'elle comprend une bague supérieure de raccordement à une coque, prolongée par un corps central dont la paroi latérale rétrécie vers le bas présente des ouvertures longitudinales et délimite une enceinte de détente du gaz communiquant avec un conduit de dispersion tronconique ménagé à l'intérieur d'un embout d'application sur la peau et débouchant à l'extérieur par un orifice d'éjection.

Selon une caractéristique spécifique de l'invention, les faces latérales des ouvertures longitudinales sont, en partie haute du corps, évasées et tournées vers l'extérieur de façon divergente et, en partie basse du corps, évasées et tournées vers l'intérieur de façon divergente.

Selon une première variante de la buse de l'invention, les ouvertures longitudinales présentent des faces latérales en vrille.

Selon une caractéristique avantageuse, la hauteur du conduit de dispersion tronconique et son diamètre sont, respectivement, inférieurs ou égaux à 10 mm.

Ce conduit débouche à l'extérieur par un orifice d'éjection présentant un décalage axial par rapport à l'axe du corps.

De préférence, ce décalage est compris entre 1,5 mm et 3,5 mm.

Selon une autre caractéristique, la hauteur de l'enceinte de détente est comprise entre 35 et 55 mm.

Selon encore une autre caractéristique, la surface totale des fentes longitudinales est comprise entre 1 080 mm² et 2 160 mm².

Selon une variante spécifique de réalisation de la buse, la section du corps central est sensiblement trapézoïdale et décroissante du haut vers le bas.

Selon une caractéristique spécifique, la paroi latérale du corps présente une inclinaison d'environ 8° par rapport à l'axe longitudinal de la buse.

Un autre objet de l'invention est un dispositif pour le traitement dermo-cosmétique des taches brunes cutanées comprenant une buse présentant les caractéristiques définies ci-dessus.

La buse de l'invention permet d'obtenir un effet cryogénique sur la peau qui est conforme à la courbe de référence correspondant à un traitement dermo-cosmétique optimal des taches brunes.

Grâce à cette buse, l'effet cryogénique se trouve concentré sur une zone cible de la peau de faible surface. Le dispositif équipé de la buse permet la mise en œuvre d'un traitement dermo-cosmétique particulièrement efficace dont les séquences sont reproductibles de façon fidèle et avec une grande précision des paramètres physico-chimiques.

Enfin, grâce à cette buse, l'effet cryogénique se trouve répartit de manière homogène sur la zone cible de la peau précédemment citée.

### BREVE DESCRIPTION DES FIGURES

D'autres caractéristiques et avantages de l'invention ressortiront à la lecture de la description qui va suivre, en référence aux figures annexées et détaillées ci-après.
La figure 1 représente une vue en perspective éclatée d'un dispositif de traitement cryogénique pourvu d'un mode de réalisation de la buse de l'invention.
Les figures 2A, 2B et 2C sont des vues, respectivement, de face, de profil et de dessus de la buse de la figure 1.
Les figures 3A, 3B et 3C sont des vues en coupe du corps de la buse des figures 1 et 2B selon trois plans A, B, C distincts et parallèles.
La figure 4 est un graphe représentant la courbe de référence de la température du traitement dermo-cosmétique mis en œuvre avec le dispositif de l'invention.
La figure 5 est une vue schématique en coupe illustrant le régime du flux de gaz dans la buse de l'invention.
La figure 6 est une vue en perspective éclatée du dispositif de traitement par cryothérapie équipé de la buse de l'invention.
Pour plus de clarté, les éléments identiques ou similaires sont repérés par des signes de référence identiques sur l'ensemble des figures.

### DESCRIPTION DÉTAILLÉE DE MODES DE RÉALISATION

Naturellement, les modes de réalisation illustrés par les figures présentées ci-dessus ne sont donnés qu'à titre d'exemples non limitatifs. Il est explicitement prévu, selon l'invention, que l'on puisse combiner entre eux ces différents modes pour en proposer d'autres.

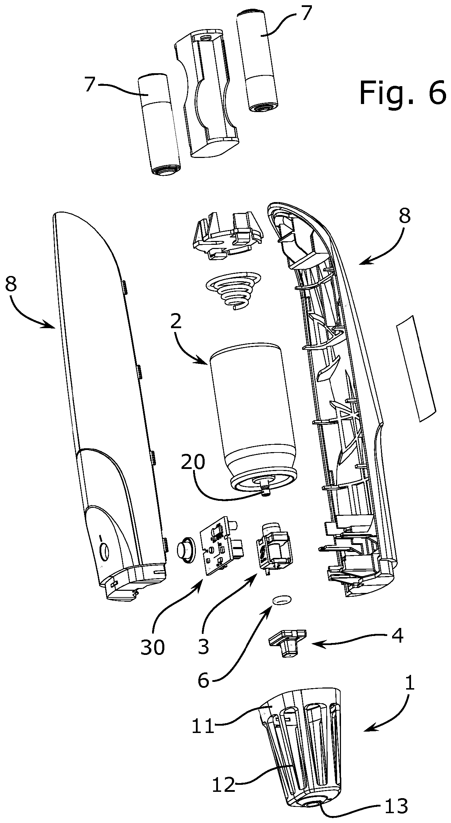

Le dispositif de traitement, tel que représenté sur la figure 6, comprend, principalement, un réservoir 2 de fluide cryogénique sous pression (à environ 6 bars), une électrovanne 3 montée sur la sortie du réservoir 2 et pilotée par un circuit électronique 30, un gicleur 4, une buse 1, un joint d'étanchéité 6 et une batterie 7 (constituée ici de deux piles). L'ensemble de ces éléments est enfermé dans un boîtier formé ici d'une coque 8 résultant de l'assemblage de deux demi-coques.

La buse 1 d'application est un des composants essentiels du dispositif de traitement des taches brunes. Elle conditionne le potentiel cryogénique du gaz et son impact sur la zone cible de la peau et influence donc directement l'efficacité clinique du traitement dermo-cosmétique.

Le fluide cryogénique est un gaz qui est en phase liquide et sous pression dans le réservoir 2. Le réservoir 2 est toujours en position ouverte et est obturé par l'électrovanne 3 montée directement sur son tube d'échappement 20.

Lorsque l'électrovanne 3 passe en position ouverte le fluide s'échappe automatiquement du réservoir 2 en direction du gicleur 4. Les phases d'ouverture et de fermeture de l'électrovanne 3 sont pilotées par le circuit électronique 30 qui gère ces séquences.

A la sortie du canal du gicleur 4, le fluide pénètre dans la buse 1 où s'opère la transition entre sa phase liquide et sa phase gazeuse (détente du gaz) jusqu'à parvenir sur la zone cible de la peau pour y produire l'effet cryogénique recherché.

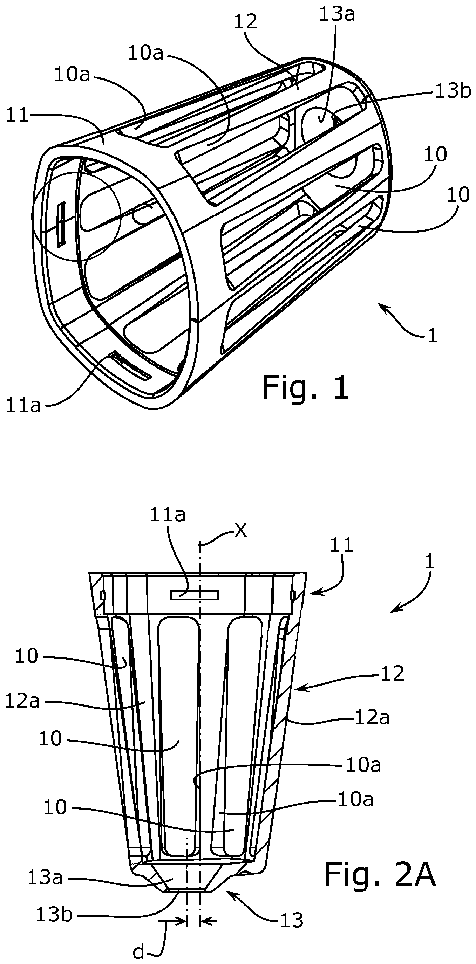

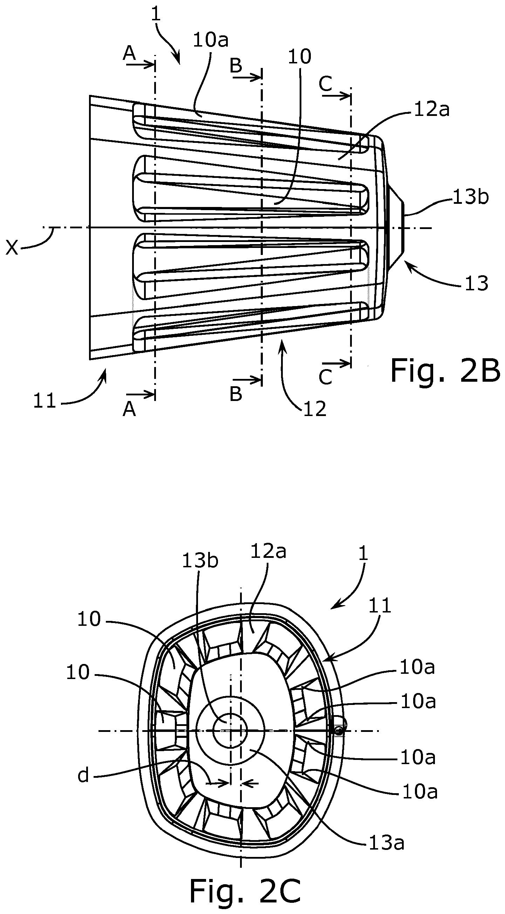

Les figures 1, 2A, 2B et 2C représentent un mode de réalisation préférentiel de la buse 1 de l'invention.

Cette buse 1, d'axe longitudinal X, comprend une bague supérieure 11 assurant son raccordement et sa fixation irréversible à la coque 8. A cet effet, la bague 11 comporte des organes de verrouillage (par exemple par encliquetage) ici sous forme de deux orifices 11a destinés à recevoir des picots complémentaires (non représentés) portés par la base de la coque 8.

Dans le mode de réalisation représenté sur les figures, la bague 11 se prolonge en partie inférieure par un corps central 12 de section sensiblement trapézoïdale dont la paroi latérale 12a est inclinée en se rétrécissant vers le bas avec un angle d'environ 8° par rapport à l'axe longitudinal de la buse 1.

Il serait possible toutefois, sans sortir du cadre de l'invention, de réaliser le corps 12 sous forme cylindrique.

La paroi 12a du corps 12, qui présente localement des ouvertures longitudinales traversantes 10, délimite intérieurement une enceinte de détente du gaz cryogénique. Les ouvertures 10 assurent, notamment, une communication de cette enceinte avec le milieu extérieur environnant qui est à température ambiante et pression atmosphérique.

Selon une variante préférentielle, les ouvertures longitudinales 10 sont constituées d'une série de fentes, et ici de 9 fentes, ayant chacune une hauteur de 40 mm et une largeur comprise entre 6 mm et 7 mm et, de préférence, égale à 6,7 mm. Ces fentes sont délimitées par des bords ou faces latérales 10a ménagées dans la paroi 12a du corps 12 et dont le profil est généralement biseauté en étant vrillé sur leur hauteur.

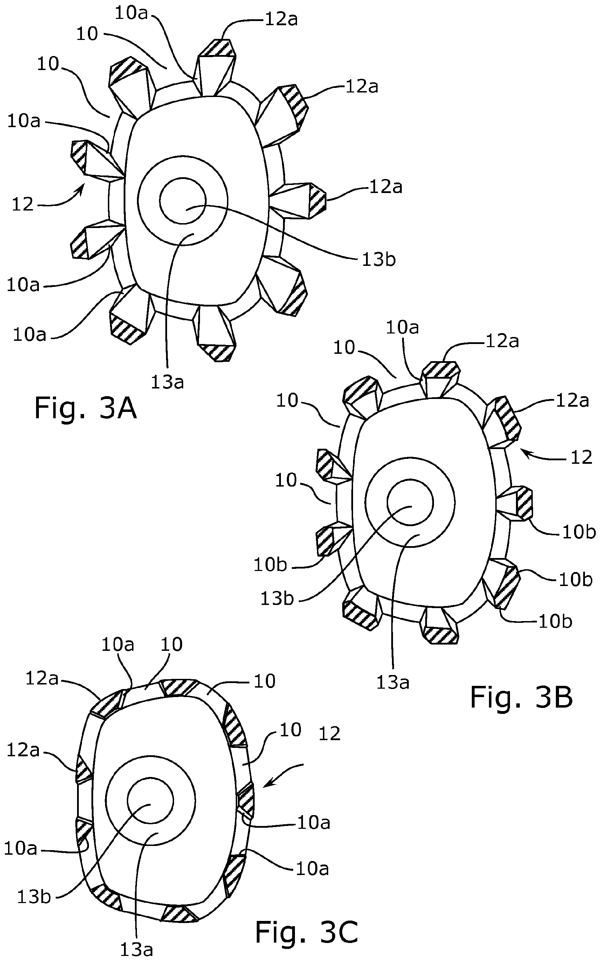

Plus précisément et comme illustré par les figures 2B, 2C et 3A à 3C, sur chaque fente 10, les faces 10a en vis-à-vis sont tournées en s'évasant, d'une part, vers l'extérieur en partie haute du corps 12 (figure 3A) et, d'autre part, vers l'intérieur en partie basse (figure 3C). La ligne d'inversion 10b de l'orientation de ces faces est située sensiblement à mi-hauteur sur les fentes 10, c'est-à-dire au niveau du plan de coupe de la figure 3B où les faces 10a sont alors localement parallèles.

Grâce à ce profil spécifique (dont les fonctions et l'influence sur le flux de gaz cryogénique seront décrits plus en détails ci-après), les ouvertures 10 contribuent à renforcer le flux de gaz laminaire tout en permettant au gaz de se détendre de façon homogène à l'intérieur de l'enceinte du corps 12. Cette détente amène le gaz à la pression atmosphérique et a donc un impact sur son potentiel cryogénique et son efficacité dermo-cosmétique. La surface d'ouverture du corps 12 ainsi que le profil et la géométrie des ouvertures 10, sont des paramètres essentiels pour optimiser les propriétés cryogéniques du gaz, comme le démontrent plus loin les essais réalisés.

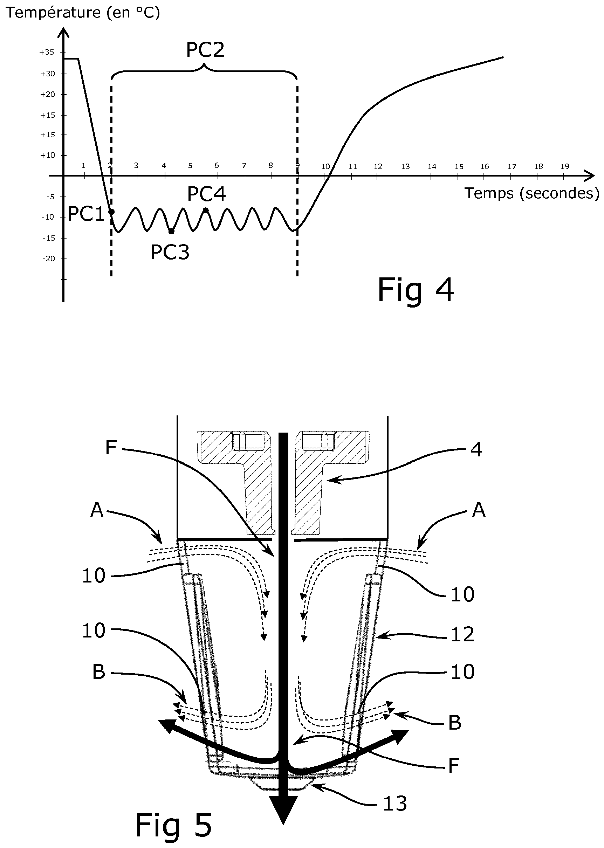

En effet, le gaz qui se trouve en phase liquide dans le réservoir 2 ne peut pas se détendre dès la sortie de l'électrovanne 3. Ce gaz arrive donc au travers du joint d'étanchéité 6 dans le gicleur 4, en étant toujours à l'état liquide. Ce n'est qu'à la sortie du gicleur 4, qu'il passe de la phase liquide à la phase gazeuse en subissant une détente. La pression tout au long de ce parcours puis lors de la transformation de la phase liquide en phase gazeuse, engendre la création d'un flux de gaz laminaire à la sortie du gicleur 4, comme illustré par la figure 5.

Le gicleur 4 est constitué d'un premier canal de 1 mm de diamètre sur une longueur de 9 mm dans sa partie initiale, puis d'un second canal de 0,1 mm de long pour un diamètre de 0,3 mm dans sa partie finale. La configuration de l'orifice de sortie du gicleur 4, avec des parois perpendiculaires à l'axe du canal de sortie, et celle du canal de sortie lui-même, induisent un flux laminaire F de fluide puisque l'angle de déviation du flux en sortie est très faible (moins de 15°). De plus, la vitesse du fluide étant déterminée par sa pression dans le réservoir 2 et les diamètres variables des différents composants qu'il traverse, celle-ci est relativement faible à la sortie du gicleur 4, ce qui est une autre caractéristique des flux laminaires (voir figure 5).

L'obtention d'un flux laminaire sur la zone cible est un objectif important de l'invention, or le gaz doit se détendre dans un volume donné en établissant un équilibre de pression avec l'air extérieur (sous pression atmosphérique).

Toutefois, cette détente ne doit pas interférer avec les régimes de flux laminaire F issu du gicleur 4 afin que le gaz puisse arriver avec un fort potentiel cinétique et cryogénique homogène sur sa cible, c'est-à-dire sur la zone destinée à subir le traitement dermo-cosmétique. En effet, s'il devait atteindre sa cible en étant en régime turbulent, l'effet cryogénique produit ne serait ni reproductible, ni homogène sur la totalité de la surface de la zone concernée.

Constatant à partir des essais une très forte reproductibilité du modèle expérimental ainsi que l'homogénéité de l'effet cryogénique sur la surface de la cible considérée, l'invention vise à l'obtention d'un régime laminaire stable du gaz en sortie de l'embout 13.

La structure spécifique de la buse 1 de l'invention permet au gaz, au moins dans la partie centrale de son volume, c'est-à-dire dans l'enceinte délimitée par le corps central 12 (correspondant à une zone théorique de forme cylindrique de 10 mm de diamètre), de générer le régime laminaire recherché.

Cependant, on observe des turbulences de part et d'autre de l'axe central X du corps 12 qui sont la conséquence de deux facteurs. Tout d'abord, le flux laminaire F associé à la détente du gaz a tendance à repousser et à déplacer, en direction de l'embout 13 et vers l'extérieur, au moins une partie du volume d'air B contenu à l'intérieur du corps 12. Conjointement, un phénomène inverse se produit du fait que le flux laminaire F issu du gicleur 4 et pénétrant dans le corps 12, provoque une dépression en partie haute qui engendre une aspiration de l'air extérieur A vers l'intérieur de la buse par effet Venturi (figure 5).

Or, la concomitance de ces deux phénomènes d'effets inverses est susceptible de créer des turbulences et de modifier notablement le caractère laminaire du flux de gaz à l'intérieur de la buse.

Plus précisément, dans la partie supérieure du corps 12, la dépression se traduisant par une aspiration de l'air extérieur A, le régime laminaire F se trouve perturbé par l'air entrant dans l'enceinte du corps 12 via les ouvertures 10.

De plus, dans la partie inférieure, le flux laminaire F a tendance à pousser le volume d'air occlus B et se trouve alors perturbé par les turbulences qui en résultent, avant même son entrée dans l'embout 13 et son arrivée sur la zone cible.

En conséquence, on assiste à une perte de l'homogénéité et de la reproductibilité des courbes de températures obtenues.

Ce sont ces phénomènes que l'invention cherche à contrôler et à maîtriser grâce au profil spécifique des ouvertures 10. Ainsi, les faces latérales 10a des ouvertures 10 forment des rampes hélicoïdales en vis-à-vis et orientées en sens inversées qui facilitent les entrées et sorties d'air, respectivement, en partie haute et en partie basse du corps 12.

Les ouvertures 10 du corps 12 influent favorablement sur le régime du flux de gaz et de plusieurs manières, toutes concourant au même effet, à savoir, maintenir et renforcer le flux laminaire F de gaz jusqu'à la zone cible tout en favorisant sa détente et en limitant les turbulences qui pourraient perturber ce flux laminaire.

Dans cet objectif, l'invention prévoit que dans la partie supérieure du corps 12, les faces latérales 10a en vis à vis des ouvertures 10 sont évasées en étant tournées vers l'extérieur de façon divergente pour favoriser l'entrée de l'air A aspiré par le flux F de gaz laminaire, comme illustré par la figure 5.

A l'inverse, dans la partie inférieure du corps 12, les faces latérale 12a en vis-à-vis des ouvertures 10 sont évasées et tournées vers l'intérieur de façon divergente pour faciliter le refoulement de l'air B repoussé par le flux laminaire F et éloigner les turbulences, comme illustré également par la figure 5

Cette configuration permet, en outre, de concentrer l'effet cryogénique en limitant les échanges de températures (de masses d'air) entre l'intérieur et l'extérieur de la buse.

En résumé, la configuration spécifique des ouvertures 10 du corps 12 de la buse permet d'obtenir un effet cryogénique reproductible et homogène sur la surface cible considérée en raison :
- d'un maintien sur une longue distance (de la sortie du gicleur à la surface de la zone cible) d'un flux laminaire;
- de la limitation de l'influence des turbulences créées en périphérie de ce flux par les phénomènes de détente et d'effet Venturi;
- de la concentration et de l'augmentation de la puissance de l'effet cryogénique au niveau de l'embout 13 de la buse 1 grâce au renforcement du caractère laminaire du flux du gaz et en évitant sa transition en régime tourbillonnaire;

Comme illustré par les figures, l'enceinte de détente du gaz située à l'intérieur du corps 12 communique, à sa partie inférieure, avec un conduit de dispersion 13a à profil tronconique, ménagé à l'intérieur d'un embout 13, et débouchant à l'extérieur par un orifice d'éjection 13b. L'embout 13 qui est situé à l'extrémité inférieure de la buse 1 est destiné à être appliqué sur la zone de la peau où se trouvent la ou les taches brunes à traiter.

L'embout 13 et, en particulier, l'orifice d'éjection 13b, présentent un décalage axial d par rapport à l'axe du corps 12 d'environ 3,80mm, comme illustré par les figures 2A et 2C.

L'embout 13 permet de produire l'effet cryogénique sur une faible surface, en y concentrant l'effet cryogénique via son orifice d'éjection central 13b dont le diamètre qui est inférieur ou égal à 10mm et est, de préférence, de 6mm (voir les essais plus loin), est situé à la sortie du conduit d'éjection 13a. Le diamètre de l'orifice 13b est déterminé de façon à pouvoir appliquer la buse 1 sur la zone cutanée à traiter dont la surface est sensiblement identique à la section de l'orifice et à concentrer l'effet cryogénique de façon uniforme tout en le limitant sélectivement à cette zone cible.

La courbe de température de référence illustrée par la figure 4 représente les variations de la température mesurée au niveau de la zone cible de la peau par unité de temps.

On entend par zone cible ou cible la surface sur laquelle est positionné l'embout 13 de la buse 1 et qui sur laquelle doit être appliqué l'effet cryogénique produit par le dispositif de traitement. La température est mesurée en degrés Celsius et les durées sont mesurées en secondes. Les temps sont mesurés à partir d'un temps noté t0 qui correspond au moment où l'électrovanne s'ouvre pour libérer le gaz liquide sous pression. Le temps t0 correspond également au démarrage de la séquence de traitement.

Cette séquence comprend d'abord une descente rapide de la température à partir de la température initiale moyenne de l'épiderme (environ 34°C) pendant une durée inférieure à 1 seconde à partir de l'ouverture de l'électrovanne (t0). Cette baisse de température permet d'atteindre un palier de températures négatives où la température reste variable par oscillations mais est maintenue dans une plage comprise entre -15°C et -5°C pendant une durée d'au moins 7 secondes et d'au plus 10 secondes. Ce palier correspond à la phase active du traitement dermo-cosmétique des taches brunes. La phase suivante consiste en une remontée progressive de la température jusqu'à sa valeur de départ.

Les paramètres de la courbe de référence, mesurés au niveau de la zone cible de l'épiderme, sont les suivants (les temps sont donnés par rapport à t0 correspondant à l'ouverture de l'électrovanne à un moment où la température initiale de la peau T0 est voisine de 34°C) :
- (PC0) est l'origine de la courbe à t0 et T0
- (PC1) est le point de la courbe de référence correspondant à la température T1 atteinte au temps t1 d'au plus 2 secondes,
- (PC2) est le point de la courbe de référence à t2 ; t2-t1 étant la durée comprise entre 7 et 10 secondes pendant laquelle la température est comprise entre - 15°C et -5°C tout en pouvant varier dans cette plage,
- (PC3) est le point correspondant au seuil (valeur minimale) de température enregistrée sur la partie de la courbe entre t1 et t2,
- (PC4) est le point correspondant au plafond (valeur maximale) de température enregistrée sur la partie de la courbe entre t1 et t2,
- (PC5) est le point où la température de la zone cible est de 0°C à t3 = 10 secondes au cours de la remontée progressive de la température.

Cette courbe de référence correspond au mode de mise en œuvre optimal du procédé de traitement qui comprend la génération d'un choc thermique puis osmotique destiné à détruire les mélanocytes sur la zone cible de l'épiderme et du derme tout en préservant les autres cellules.

Cette courbe de référence est obtenue et réalisée de manière reproductible et homogène sur l'ensemble de la zone à traiter si le gaz passe d'une phase liquide à une phase gazeuse avant d'arriver sur la zone cible où elle induit l'effet cryogénique. La structure de la buse 1 conditionne l'apparition de ce phénomène et est donc critique à cet égard. En effet, le transit du flux gazeux au travers de la buse 1 implique le passage très critique d'une phase liquide à une phase gazeuse. Or, ce changement d'état est influencé par plusieurs paramètres, dont ;
- la surface (S) d'ouverture des ouvertures ou fentes 10 ;
- la distance (D) entre la sortie du gicleur et la zone cible sur laquelle l'effet cryogénique a vocation à s'exercer et ;
- la hauteur (H) de l'embout 13 de la buse 1.

En effet, si la distance (D) et/ou la surface (S) d'ouverture des fentes 10 est/sont trop faible(s), le gaz n'a pas la possibilité de se détendre et est projeté sur la cible sous la forme d'un aérosol constitué d'un mélange de gouttelettes liquides et de gaz. La phase liquide s'accumule ainsi sur la cible, s'étend en dehors de la cible sur des zones qui ne doivent pas être traitées, se détend et s'évapore progressivement en engendrant ainsi des variations de températures incontrôlées, non reproductibles et non homogènes sur l'ensemble de la zone à traiter. Dans le cas inverse, le gaz se disperse et ne peut mettre en œuvre tout son potentiel cryogénique. Dans ces deux situations, la courbe de température obtenue ne correspond pas à la courbe de référence et les résultats du traitement ne sont donc pas satisfaisants.

Par ailleurs, en présence de l'humidité résiduelle environnante, l'effet cryogénique engendre, sur la peau, la formation d'eau cristallisée en glace (givre), ce qui permet de maintenir stable une température négative pendant une durée déterminée.

Or, une forme trop plate de la buse 1 (hauteur H faible) ne permet pas de maintenir assez longtemps la température dans une plage comprise entre -15°C et -5°C alors qu'une forme trop profonde (hauteur H importante) engendre une durée trop longue de contact entre la cible et l'eau cristallisée, engendrant alors des températures trop basses. L'invention vise à sélectionner un profil spécifique de la buse permettant d'obtenir une courbe de température correspondant de façon fidèle à la courbe optimale de référence.

Les expérimentations et essais pratiqués et commentés ci-après ont pour objectif de vérifier et de préciser l'influence des différents paramètres (S), (D) et (H) sur les courbes de température obtenues au niveau de la zone cible.

Dans ces expérimentations, les températures sont exprimées en degrés Celsius et les temps en secondes. Les chiffres en gras dans les tableaux indiquent que les valeurs indiquées sont conformes aux valeurs ou aux plages de valeurs de la courbe de référence.

L'origine des temps (t0) correspond au moment où démarre l'activation du circuit électronique 30 et, en particulier, la commande de l'électrovanne 3. Pour des raisons techniques, cette séquence démarre par une phase de repos de 0,5 secondes, avant que le premier signal soit envoyé à l'électrovanne 3 pour la commuter en position ouverte et libérer ainsi le gaz du réservoir 2.

En effet, dans le dispositif de traitement de l'invention, le réservoir 2 de gaz, maintenu en position ouverte, est raccordé à l'électrovanne 3 qui se trouve au repos en position fermée. Lorsque l'électrovanne 3 est alimentée en courant via le circuit électronique 30 (séquence programmée), elle passe d'une position fermée à une position ouverte en laissant le gaz s'échapper. L'arrêt du circuit électronique 30 entraîne le retour à la situation de repos pour l'électrovanne 3. Une séquence est ainsi constituée d'une suite de signaux permettant la commutation en position ouverte ou fermée de l'électrovanne selon un ordre très précis et selon des durées spécifiques très précises. La suite de commutations de durées déterminées (et variables d'une commutation à une autre), espacées selon des durée précises correspond au protocole de mise en œuvre clinique du traitement dermo-cosmétique.

### ESSAI 1 - INFLUENCE DE LA HAUTEUR (H) DE L'EMBOUT 13 SUR LA COURBE DES TEMPERATURES

L'objectif de cet essai est de réaliser différentes mesures des températures en fonction du temps tout en faisant varier la hauteur H de l'embout 13 de la buse entre 0 et 4,12 mm.

Les constations sont les suivantes. La hauteur H de l'embout modifie peu la forme de la courbe cryogénique. En revanche, l'absence d'embout (H = 0 mm) ne permet pas d'atteindre des températures inférieures à -15°C rapidement et les oscillations ne sont pas présentes. En revanche, pour des embouts de hauteur 4,2 mm 5,6 mm et 6,2 mm, les variations s'observent surtout dans phase de remontée en température mais ne sont toutefois pas significatives.

**Tableau 1 - Valeurs des paramètres de la courbe de référence pour différentes hauteurs H de l'embout (0 mm ≤ H ≤ 6,2 mm)**

| POINTS CRITIQUES | REFERENCES | 4,2 mm | 0 mm : | 5,6 mm | 6,2 mm |
|---|---|---|---|---|---|
| PC1 | ≤ -5°C | **-6,56** | **-6,88** | **-7,01** | **-7,18** |
| PC2 | ≥ 7 sec. | **17,70** | **18,54** | **15,23** | **13,62** |
| PC3 | ≥ -15°C (+/-5%) | **-14,84** | **-14,02** | -15,79 | -16,55 |
| PC4 | ≤-5°C (+/-5%) | **-6,55** | **-6,88** | **-7,01** | **-7,16** |

Seules les hauteurs 0 ≥ H ≥ 4,2 mm, associées à une séquence spécifique et à un profil de buse spécifique, permettent de respecter les valeurs de référence.

En adaptant soit la séquence, soit la forme de la buse 1, soit le diamètre du canal du gicleur, il est possible de considérer qu'une plage de hauteurs comprises entre 0 et 10 mm et, de préférence, inférieure ou égale à 6 mm, permette d'obtenir un effet cryogénique conforme aux valeurs de la courbe de référence.

### ESSAI 2 - INFLUENCE DE LA DISTANCE (D) ENTRE LA SORTIE DU GICLEUR ET LA ZONE CIBLE SUR LES COURBES DE TEMPERATURE

L'objectif de cet essai est de réaliser différentes mesures des températures en fonction du temps tout en faisant varier la distance (D) entre la sortie du gicleur et la cible entre 25 mm et 65 mm.

Les constations sont les suivantes. Des distances (D) faibles et, en particulier, inférieures à 45 mm, ne permettent pas au fluide cryogénique de se détendre de façon satisfaisante. Cette situation induit des perturbations dans les mesures effectuées, puisqu'il y a formation au niveau de la cible de gouttelettes de fluide (liquide). Les courbes de températures obtenues présentent des oscillations d'amplitudes très importantes correspondant alors aux projections du liquide. Pour une courbe donnée, aucune des valeurs de référence des paramètres n'est respectée (les valeurs conformes étant en gras).

**Tableau 2 - Valeurs des paramètres de la courbe de référence pour différentes distances D (25 mm ≤ D ≤ 40 mm).**

| POINTS CRITIQUES | REFERENCES | 25 mm | 30 mm | 35 mm | 40 mm |
|---|---|---|---|---|---|
| PC1 | ≤ -5°C | **-6,32** | -1,64 | -3,92 | -3,49 |
| PC2 | ≥ 7 sec. | 4,51 | **8,00** | **10,61** | **10,59** |
| PC3 | ≥ -15°C (+/-5%) | -17,75 | -18,64 | -22,72 | -18,43 |
| PC4 | ≤ -5°C (+/-5%) | 4,26 | -1,64 | -3,92 | -3,49 |

Des distances (D) importantes, en particulier, supérieures ou égales à 45 mm permettent au gaz de se détendre et d'exercer son potentiel cryogénique sans formation de gouttelettes, avec cependant une baisse de ce potentiel à mesure que l'on s'éloigne de la cible. Pour la plupart des distances expérimentées, les valeurs des paramètres de la courbe de référence ne sont pas respectées (valeurs conformes en vert), sauf pour les courbes correspondant aux distances D = 45 mm et D = 55 mm.

**Tableau 3 - Valeurs des paramètres de la courbe de référence pour différentes distances D (45 mm ≤ D ≤ 65 mm)**

| POINTS CRITIQUES | REFERENCES | 45 mm | 50 mm | 55 mm | 60 mm | 65 mm |
|---|---|---|---|---|---|---|
| PC1 | ≤ -5°C | **-6,69** | **-7,86** | **-8,84** | **-5,34** | -1,07 |
| PC2 | ≥ 7 sec. | **13,38** | **10,76** | **8,64** | 5,93 | 0,00 |
| PC3 | ≥ -15°C (+/-5%) | **-15,55** | -16,26 | **-14,92** | **-8,52** | -3,60 |
| PC4 | ≤ -5°C (+/-5%) | **-6,64** | **-7,86** | **-7,24** | -1,61 | 5,42 |

Par conséquent, seules les distances D = 45 mm et D = 55 mm séparant la sortie du gicleur de la cible, associées à une séquence de pulvérisation spécifique et à un gicleur spécifique, permettent de respecter ces valeurs.

Ainsi, en adaptant soit la séquence, soit le diamètre du canal du gicleur, il est possible de considérer qu'une plage de distances (D) comprises entre 35 mm et 55 mm permette d'obtenir un effet cryogénique conforme aux valeurs des paramètres de la courbe de référence.

### ESSAI 3 - INFLUENCE DE LA SURFACE (S) D'OUVERTURE DE LA BUSE SUR LES COURBES DE TEMPERATURE

L'objectif de cet essai est de réaliser différentes mesures de température en fonction du temps tout en faisant varier la surface (S) des fentes 10 du corps 12 de la buse 1 et en faisant varier ainsi, dans la direction horizontale ou verticale, la surface d'échange avec l'air ambiant contribuant à la détente du gaz et au renforcement du flux laminaire.

Une modification verticale consiste à réduire plus ou moins la hauteur des fentes en diminuant ainsi leur surface d'échange, par rapport à une buse de référence présentant une surface globale d'ouverture de 2 160 mm², dans le sens de la sortie du gaz vers la cible. Une modification horizontale consiste à réduire plus ou moins la largeur des fentes, c'est-à-dire à les fermer perpendiculairement au sens de sortie du gaz vers la cible.

Les constations sont les suivantes. Le corps 12 de la buse 1 possédant des fentes 10 selon la configuration spécifique de l'invention et telle qu'illustrée par les figures, permet d'obtenir une détente complète du gaz et d'utiliser au maximum son potentiel cryogénique.

En revanche, une fermeture totale de la buse engendre une courbe non conforme à la courbe de référence, car la détente du gaz est impossible du fait de l'absence d'équilibre, dans un temps donné, de la pression du gaz avec la pression atmosphérique environnante.

**Tableau 4 - Valeurs des paramètres des courbes avec un corps de buse sans ouverture (0% d'ouverture) et un corps de buse avec 9 fentes de surface totale d'ouverture de 2 160 mm2 (100% d'ouverture).**

| POINTS CRITIQUES | REFERENCES | 0 mm² 0% | 2 160 mm² 100% |
|---|---|---|---|
| PC1 | ≤ -5°C | -0,91 | **-7,26** |
| PC2 | ≥ 7 sec. | **16,15** | **8,00** |
| PC3 | ≥ -15°C (+/-5%) | **-13,13** | **-7,24** |
| PC4 | ≤ -5°C (+/-5%) | 2,82 | **-7,18** |

Une première observation est que, quelle que soit la séquence, la conformation du gicleur (donc le débit), la quantité de gaz administrée, la forme de la buse et une surface globale d'ouverture de cette dernière d'au moins 1.080 mm² permet à l'ensemble du gaz de se détendre et de disposer de la totalité de son potentiel cryogénique.

Une seconde observation est que plus le pourcentage d'ouverture est faible, plus les courbes s'éloignent de la courbe de référence et des valeurs de ses paramètres.

**Tableau 5 - Valeurs des paramètres des courbes pour différents modes d'ouverture verticale des fentes de la buse.**

| POINTS CRITIQUES | REFERENCES | 2 160 mm² 100% | 1 620 mm² 75% (v) | 1 080 mm² 50% (v) | 540 mm² 25% (v) |
|---|---|---|---|---|---|
| PC1 | ≤ -5°C | **-7,26** | **-6,70** | **-6,99** | -1,42 |
| PC2 | ≥ 7 sec. | **8,00** | **9,58** | **16,50** | **13,73** |
| PC3 | ≥ -15°C (+/-5%) | **-7,24** | **-8,41** | **-12,39** | **-13,13** |
| PC4 | ≤ -5°C (+/-5%) | **-7,18** | **-6,66** | **-6,86** | -0,09 |

**Tableau 6 - Valeurs des paramètres des courbes pour différents modes d'ouverture horizontale des fentes de la buse.**

| POINTS CRITIQUES | REFERENCES | 2 160 mm² 100% | 1 620 mm² 75% (h) | 1 080 mm² 50% (h) | 540 mm² 25% (h) |
|---|---|---|---|---|---|
| PC1 | ≤ -5°C | **-7,26** | **-8,43** | -1,86 | -3,10 |
| PC2 | ≥ 7 sec. | **8,00** | **12,67** | **13,88** | 4,10 |
| PC3 | ≥ -15°C (+/-5%) | **-7,24** | **-8,41** | **-12,39** | **-13,13** |
| PC4 | ≤ -5°C (+/-5%) | -7,18 | **-8,41** | -0,41 | -1,64 |

Ainsi, on remarque que l'impact sur les courbes de l'ouverture des fentes du corps de la buse est différent, pour un même pourcentage, selon qu'il s'agisse d'une ouverture verticale ou horizontale.

**Tableau 7 - Valeurs des paramètres des courbes pour un mode de d'ouverture à 50% horizontale (notée h) et à 50% verticale (notée v) de la buse.**

| POINTS CRITIQUES | REFERENCES | 2 160 mm² 100% | 1 080 mm² 50% (v) | 1 080 mm² 50% (h) |
|---|---|---|---|---|
| PC1 | ≤ -5°C | **-7,26** | **-6,99** | -1,86 |
| PC2 | ≥ 7 sec. | **8,00** | **16,50** | **13,88** |
| PC3 | ≥ -15°C (+/-5%) | **-7,24** | **-12,39** | **-12,39** |
| PC4 | ≤ -5°C (+/-5%) | **-7,18** | **-6,86** | -0,41 |

L'expérience montre que pour deux surfaces identiques (chacune de 1 080 mm²), les courbes de températures et leurs paramètres sont différentes selon que l'ouverture s'effectue dans le sens vertical ou horizontal.

En adaptant soit la séquence, soit le diamètre du canal du gicleur, il est possible de considérer qu'une ouverture globale minimum de 1 080 mm² est nécessaire pour garantir un effet cryogénique conforme à celui de la courbe de référence.

## Revendications

1. Buse pour un dispositif de traitement dermo-cosmétique des taches brunes cutanées par cryothérapie cyto-sélective comprenant un gicleur alimenté par un réservoir de gaz cryogénique liquéfié sous pression, ladite buse comprenant une bague supérieure (11) de raccordement à une coque (8), prolongée par un corps central (12) dont la paroi latérale (12a) rétrécie vers le bas présente des ouvertures longitudinales (10) et délimite une enceinte de détente du gaz communiquant avec un conduit de dispersion (13a) tronconique ménagé à l'intérieur d'un embout (13) d'application sur la peau et débouchant à l'extérieur par un orifice d'éjection (13b), **caractérisée en ce que** lesdites ouvertures longitudinales (10) de la paroi latérale (12a) présentant des faces latérales (10a) qui sont, en partie haute du corps (12), évasées et tournées vers l'extérieur de façon divergente et évasées et, en partie basse du corps (12), évasées et tournées vers l'intérieur de façon divergente.

2. Buse selon la revendication 1, **caractérisée en ce que** lesdites ouvertures longitudinales (10) présentent des faces latérales (12a) en vrille.

3. Buse selon l'une des revendications précédentes, **caractérisée en ce que** la hauteur (H) du conduit de dispersion tronconique (13a) et son diamètre sont, respectivement, inférieurs ou égaux à 10 mm.

4. Buse selon l'une des revendications précédentes, **caractérisée en ce que** ledit orifice d'éjection (13b) de l'embout (13) présente un décalage axial (d) par rapport à l'axe (X) du corps (12).

5. Buse selon la revendication précédente, **caractérisée en ce que** le décalage (d) est compris entre 1,5 mm et 3,5 mm.

6. Buse selon l'une des revendications précédentes, **caractérisée en ce que** la hauteur (D) de l'enceinte de détente est comprise entre 35 et 55 mm.

7. Buse selon l'une des revendications précédentes, **caractérisé en ce que** la surface totale (S) des ouvertures longitudinales (10) du corps (12) est comprise entre 1080 mm² et 2160 mm².

8. Buse selon l'une des revendications précédentes, **caractérisée en ce que** lesdites ouvertures longitudinales du corps (12) comprennent 9 fentes (10) identiques.

9. Buse selon l'une des revendications précédentes, **caractérisée en ce que** la section du corps central (12) est sensiblement trapézoïdale et décroissante du haut vers le bas.

10. Buse selon l'une des revendications précédentes, **caractérisée en ce que** la paroi latérale (12a) du corps (12) présente une inclinaison d'environ 8° par rapport à l'axe longitudinal (X) de la buse.

## Patentansprüche

1. Düse für eine Vorrichtung zur dermokosmetischen Behandlung von braunen Flecken auf der Haut durch zytoselektive Kryotherapie, umfassend eine Düse, die von einem Vorratsbehälter mit verflüssigtem kryogenem Gas unter Druck gespeist wird, wobei die Düse einen oberen Ring (11) zur Verbindung mit einer Schale (8) umfasst, durch einen Zentralkörper (12) verlängert wird, dessen nach unten verjüngte Seitenwand (12a) Längsöffnungen (10) aufweist und eine Gasexpansionskammer begrenzt, die mit einem kegelstumpfförmigen Verteilungskanal (13a) in Verbindung steht, der im Inneren einer Spitze (13) zum Aufbringen auf die Haut angeordnet ist und durch eine Ausstoßöffnung (13b) nach außen mündet, **dadurch gekennzeichnet, dass** die Längsöffnungen (10) der Seitenwand (12a) Seitenflächen (10a) aufweisen, die im oberen Teil des Körpers (12) aufgeweitet und auseinanderlaufend nach außen gedreht und aufgeweitet und im unteren Teil des Körpers (12) aufgeweitet und auseinanderlaufend nach innen gedreht sind.

2. Düse nach Anspruch 1, **dadurch gekennzeichnet, dass** die Längsöffnungen (10) spiralförmige Seitenflächen (12a) aufweisen.

3. Düse nach einem der vorhergehenden Ansprüche, **dadurch gekennzeichnet, dass** die Höhe (H) des kegelstumpfförmigen Verteilungskanals (13a) bzw. sein Durchmesser kleiner oder gleich 10 mm sind.

4. Düse nach einem der vorhergehenden Ansprüche, **dadurch gekennzeichnet, dass** die Ausstoßöffnung (13b) der Spitze (13) einen axialen Versatz (d) bezüglich der Achse (X) des Körpers (12) aufweist.

5. Düse nach dem vorhergehenden Anspruch, **dadurch gekennzeichnet, dass** der Versatz (d) zwischen 1,5 mm und 3,5 mm beträgt.

6. Düse nach einem der vorhergehenden Ansprüche, **dadurch gekennzeichnet, dass** die Höhe (D) der Expansionskammer zwischen 35 und 55 mm beträgt.

7. Düse nach einem der vorhergehenden Ansprüche, **dadurch gekennzeichnet, dass** die Gesamtfläche (S) der Längsöffnungen (10) des Körpers (12) zwischen 1080 mm² und 2160 mm² beträgt.

8. Düse nach einem der vorhergehenden Ansprüche, **dadurch gekennzeichnet, dass** die Längsöffnungen des Körpers (12) 9 identische Schlitze (10) umfassen.

9. Düse nach einem der vorhergehenden Ansprüche, **dadurch gekennzeichnet, dass** der Querschnitt des Zentralkörpers (12) im Wesentlichen trapezförmig ist und von oben nach unten abnimmt.

10. Düse nach einem der vorhergehenden Ansprüche, **dadurch gekennzeichnet, dass** die Seitenwand (12a) des Körpers (12) eine Neigung von etwa 8° bezogen auf die Längsachse (X) der Düse aufweist.

## Claims

1. Nozzle for a device for the dermo-cosmetic treatment of dark skin spots by cyto-selective cryotherapy comprising a spray jet supplied by a tank of pressurized liquefied cryogenic gas, said nozzle comprising an upper ring (11) for connecting to a shell (8), extended by a central body (12) of which the side wall (12a) which narrows downward has longitudinal openings (10) and delimits a gas expansion chamber communicating with a frustoconical dispersal conduit (13a) arranged inside an end piece (13) to be applied to the skin and opening to the outside through a discharge port (13b), **characterized in that** said longitudinal openings (10) in the side wall (12a) have side faces (10a) which are, in the upper part of the body (12), flared and turned outward in a divergent manner, and, in the lower part of the body (12), flared and turned inward in a divergent manner.

2. Nozzle according to claim 1, **characterized in that** said longitudinal openings (10) have sideways side faces (12a).

3. Nozzle according to either of the preceding claims, **characterized in that** the height (H) of the frustoconical dispersal conduit (13a) and its diameter are, respectively, less than or equal to 10 mm.

4. Nozzle according to any of the preceding claims, **characterized in that** said discharge port (13b) of the end piece (13) has an axial offset (d) with respect to the axis (X) of the body (12).

5. Nozzle according to the preceding claim, **characterized in that** the offset (d) is comprised between 1.5 mm and 3.5 mm.

6. Nozzle according to any of the preceding claims, **characterized in that** the height (D) of the expansion chamber is comprised between 35 and 55 mm.

7. Nozzle according to any of the preceding claims, **characterized in that** the total surface area (S) of the longitudinal openings (10) in the body (12) is comprised between 1,080 mm² and 2,160 mm².

8. Nozzle according to any of the preceding claims, **characterized in that** said longitudinal openings in the body (12) comprise 9 identical slits (10).

9. Nozzle according to any of the preceding claims, **characterized in that** the section of the central body (12) is substantially trapezoidal and decreasing from the top to the bottom.

10. Nozzle according to any of the preceding claims, **characterized in that** the side wall (12a) of the body (12) has an inclination of around 8° with respect to the longitudinal axis (X) of the nozzle.
